# EUROPEAN PATENT APPLICATION

(11) **EP 4 678 763 A1**
(43) Date of publication of application: **14.01.2026**
(21) Application number: 25188835.0
(22) Date of filing: 10.07.2025
(51) Int. Cl.: C12P 33/02, C12P 41/00

(54) **A HIGH YIELDING ENANTIOSELECTIVE ENZYMATIC PROCESS FOR PREPARING URSODEOXYCHOLIC ACID**

(30) Priority: 12.07.2024 IN 202441053660
(71) Applicant: Symbio Generrics India Private Limited, Bengaluru, Karnataka 560102 (IN)
(72) Inventor: HOLKAR, Anil Ganpatrao, 411041 Pune (IN); MANJATHURU, Mahalinga, 575019 Mangaluru (IN); CHOWDARY, Yirigineni Ramakrishna, 524312 SPSR Nellore (IN); GURUGUBELLI, Swarajya Lakshmi, 532005 Srikakulam (IN); NAIK, Dinesh Narayan, 581348 Honnavar (IN); POOJARY, Santhosha Sheena, 576230 Kundapur Taluk (IN)
(74) Representative: FRKelly

(57) **Abstract**

Improved enantioselective enzymatic processes for preparing ursodeoxycholic acid with high pharmaceutical purity are provided. Also provided are improved enantioselective enzymatic processes for preparing ursodeoxycholic acid with high yield and high pharmaceutical and enantiomeric purity.

## Description

This patent application claims the benefit of priority from Indian Provisional Application Serial No. 202441053660 filed July 12, 2024, teachings of which are incorporated herein by reference in their entirety.

### FIELD OF INVENTION

The present invention relates to an improved enantioselective enzymatic process for preparing ursodeoxycholic acid with high pharmaceutical purity. The present invention also covers an improved enantioselective enzymatic process for preparing ursodeoxycholic acid with high yield and high pharmaceutical and enantiomeric purity.

### BACKGROUND OF THE INVENTION

Bile acids, essential biomolecules, are necessary for digesting and absorbing fats, fatty acids, and hydrophobic vitamins. Ursodeoxycholic acid (UDCA), a bile acid present in humans only in minimal quantities, has recently garnered significant therapeutic value for its role in dissolving gallstones composed of cholesterol,

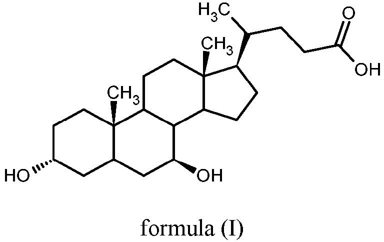

Ursodeoxycholic acid (UDCA) and its diastereomer chenodeoxycholic acid (CDCA) have, been employed for many years for the medical treatment of gallstone complaints. The two compounds differ merely by the configuration of the hydroxyl group on C atom 7 (UDCA: β-configuration, CDCA: α-configuration). To prepare UDCA, a variety of processes are described in the prior art, and these processes are carried out by purely chemical means or else as a combination of chemical and enzymatic process steps. The starting point is, in each case, cholic acid (CA), or CDCA, or 7- KLCA which is prepared starting from cholic acid or from animal source.

An important precursor for the synthesis of UDCA is 12-ketoursodeoxycholic acid, which can be converted into UDCA by a Wolff-Kishner reduction. A route, described in the literature, for the synthesis of 12-ketoursodeoxycholic acid starts with cholic acid (3α, 7α, 12α-trihydroxy-5β-cholanic acid), which may be prepared by two oxidative steps which are catalyzed by 7α- and 12α-HSDHs, and one reductive step, catalyzed by a 7β-HSDH disclosed in Bovara R et al. (1996) A new enzymatic route to the synthesis of 12-ketoursodeoxycholic acid. Biotechnol. Lett. 18:305-308; and Monti D et al. (2009) One-pot multienzymatic synthesis of 12-ketoursodeoxycholic acid: Subtle cofactor specificities rule the reaction equilibria of five biocatalysts working in a row. Adv. Synth. Catal. 351: 1303-1311. Bovara et al. (1996) reported an enzymatic route which presents several drawbacks, including the use of enzymes with mismatched cofactor requirements [Nicotinamide Adenine Dinucleotide (NAD+) versus Nicotinamide Adenine Dinucleotide Phosphate (NADP+)], which complicates in situ cofactor regeneration and hinders the development of an efficient, integrated biocatalytic process. Additionally, the reversibility of dehydrogenase-catalyzed reactions necessitates tight control over reaction equilibria, while the limited stability and reusability of the enzymes further restrict process scalability.

Monti et al. (2009) later expanded upon this concept by introducing a one-pot, multienzyme cascade comprising five different hydroxysteroid dehydrogenases to carry out a series of selective oxidations and reductions leading to 12-KUDCA. Although this approach demonstrates improved conversion through enzymatic synergy, it also suffers from key limitations. The cascade involves enzymes with subtle and overlapping cofactor specificities [e.g., NAD+, Nicotinamide Adenine Dinucleotide Hydrogen (NADH), NADP+, Nicotinamide Adenine Dinucleotide Phosphate Hydrogen (NADPH)], making cofactor recycling highly challenging. Moreover, the differing biochemical requirements and stabilities of the enzymes-sourced from various microbial origins-introduce incompatibilities in a unified reaction environment. Reaction equilibria are also strongly influenced by the thermodynamics of individual enzymatic steps, often necessitating the use of additional driving forces or reaction engineering to push the process forward.

None of these prior arts provide a high yielding enantioselective enzymatic process for preparing ursodeoxycholic acid with high enantiomeric purity. Accordingly, there remains a need for an improved biocatalytic system for the synthesis of ursodeoxycholic acid that overcomes the limitations of cofactor incompatibility, enzyme instability, reversibility of reactions, and overall process complexity, while enabling efficient transformation under scalable and industrially relevant conditions.

### SUMMARY OF THE INVENTION

The present invention relates to an improved enantioselective enzymatic process for preparing ursodeoxycholic acid of Formula (I).

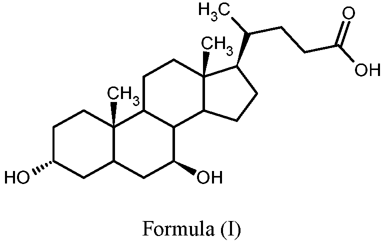

The present invention further relates to an improved high-yielding enantioselective enzymatic process for preparing ursodeoxycholic acid of Formula (I) with high enantiomeric purity.

A first aspect of the present invention provides a process for the preparation of the intermediate of Formula (III), useful for the preparation of ursodeoxycholic acid of Formula (I), 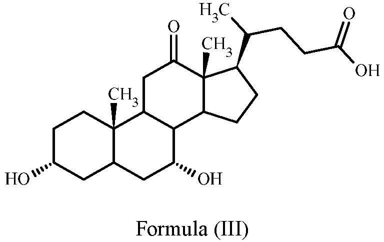 comprising enantioselective oxidation of cholic acid of formula (II) 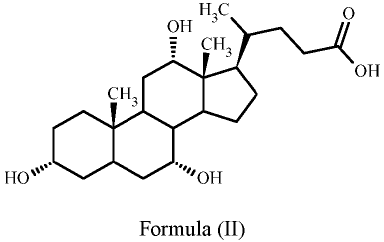 in the presence of suitable enzymes.

A second aspect of the present invention provides a process for the preparation of the intermediate of Formula (IV), useful for the preparation of ursodeoxycholic acid of Formula (I)., 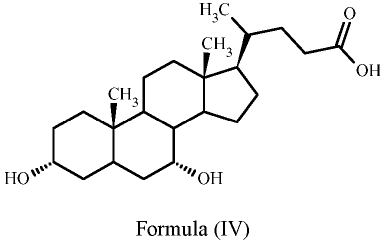 comprising base catalysed reduction of the compound of formula (III).

A third aspect of the present invention provides a process for the preparation of the intermediate of Formula (V), useful for the preparation of ursodeoxycholic acid of Formula (I)., 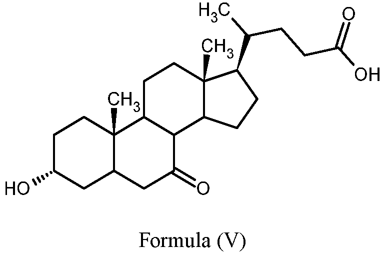 comprising enantioselective oxidation of the compound of formula (IV) in the presence of suitable enzymes.

A fourth aspect of the present invention provides a process for the preparation of ursodeoxycholic acid of Formula (I) in crude form 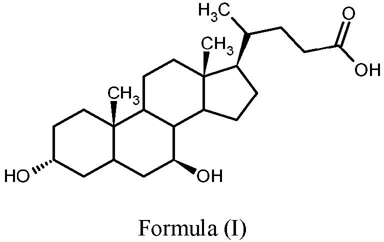 comprising enantioselective reduction of compound of formula (V) in the presence of suitable enzymes.

A fifth aspect of the present invention provides a process for the preparation of ursodeoxycholic acid of Formula (I) in a pharmaceutically pure form 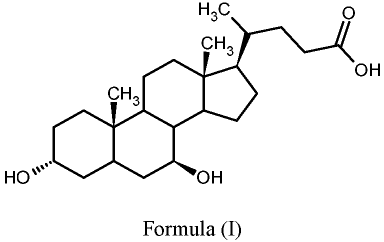 comprising treatment of crude ursodeoxycholic acid of Formula (I) with a base followed by an acid.

A sixth aspect of the present invention provides a process for the preparation of ursodeoxycholic acid of Formula (I) in an enantiomerically pure form and meeting USP, EP, JP, IP Pharmacopoeia specifications 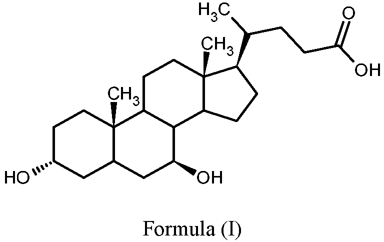 comprising treatment of crude ursodeoxycholic acid of Formula (I) with a base followed by an acid.

A seventh aspect of the present invention provides a process for the preparation of ursodeoxycholic acid of Formula (I) in pharmaceutically and enantiomerically pure form 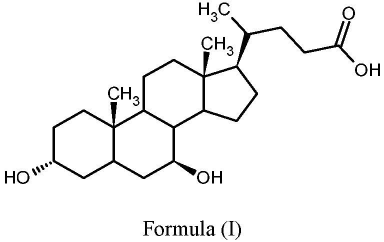 comprising treatment of crude ursodeoxycholic acid of Formula (I) with a suitable base followed by an acid.

An eight aspect of the present invention provides a process for the preparation of ursodeoxycholic acid of Formula (I) in crude form 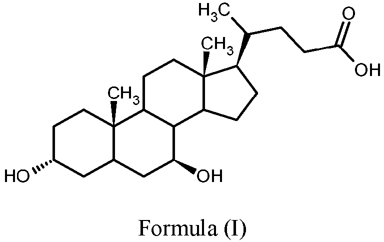 comprising enantioselective reduction of the compound of formula (V) in the presence of at least two suitable enzymes.

### BRIEF DESCRIPTION OF THE DRAWINGS

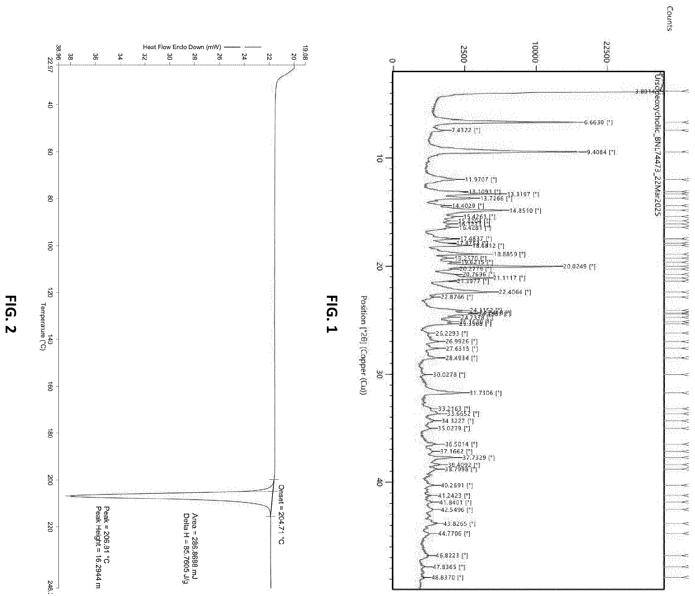

Fig.1 depicts the Powder XRD pattern of the highly enantiomerically pure crystalline Ursodeoxycholic acid.
Fig.2 depicts the DSC thermogram of the highly enantiomerically pure crystalline Ursodeoxycholic acid.

### DETAILED DESCRIPTION OF THE INVENTION

Hereinafter, preferred modes for carrying out the present invention are described. The embodiments outlined below are provided solely for illustrative purposes, to exemplify typical implementations of the present invention. These embodiments are not intended to limit the scope of the invention in any way.

As used herein, the singular forms "a," "an," and "the" are intended to include plural forms as well, unless the context explicitly indicates otherwise.

The terms "includes," "comprises," "including," and/or "comprising," and variations thereof, are intended to denote non-exclusive inclusion. That is, a composition, device, or method that includes a list of elements or steps does not necessarily consist solely of those listed elements or steps. It may also include additional elements, features, operations, or steps that are not expressly mentioned or are inherent to such a composition, device, or method. In other words, the presence of one or more components in a system or method described using the term "comprises" does not exclude the existence of other components or elements not explicitly stated.

As used herein, the term "and/or" encompasses any and all possible combinations and arrangements of one or more of the associated listed items.

Unless otherwise specified, all terms (including scientific and technical terms) used in the present disclosure shall be interpreted as understood by one of ordinary skill in the relevant art. Terms that are not defined herein should be construed in accordance with their standard usage in the relevant field and should not be interpreted in an unduly narrow or overly formal manner unless clearly indicated otherwise.

The enantioselective oxidation of cholic acid of Formula (II), is carried out in presence of one or more enzymes to obtain a compound of Formula (III). The enzyme may exist in the form of powder or suspension. The powder may be in the spray dried form

The aqueous portion of the reaction mixture in which the enantioselective oxidation of cholic acid of Formula (II) proceeds, preferably contains a buffer, e.g., a dipotassium hydrogen phosphate, potassium phosphate, HCl or triethanolamine buffer or the like.

The pH of the reaction mass for the enantioselective oxidation of cholic acid of Formula (II) may be adjusted using base such as sodium hydroxide, Sodium Bicarbonate (NaHCO₃), Potassium Bicarbonate (KHCO₃), Ammonium Bicarbonate, Ammonia Solution and the like. Most preferable the base is sodium hydroxide.

The pH of the reaction mass for the enantioselective oxidation of cholic acid of Formula (II) may be adjusted between 7 to 12. More preferably the pH of the reaction is reaction mass is between 8 to 10, most preferably, it is between 8.5 to 9.5.

The temperature of the reaction mass for the enantioselective oxidation of cholic acid of Formula (II) may be between 40 to 100°C. More preferably it is between 50 to 90°C. Most preferably it is between 70 to 80°C.

The enantioselective oxidation of cholic acid of Formula (II), may be carried out in a suitable solvent. Suitable solvent may be selected from the group comprising of water; alcohols, such as methanol, ethanol and isopropanol, 2-methoxyethanol, 2-ethoxyethanol, 2-methoxypropanol, 3-methoxypropanol, 3-ethoxypropanol, and 3-ethoxypropanol.

The enantioselective oxidation of cholic acid of Formula (II), may be carried out in presence of a cofactor such as NAD.

The reduction of a compound of Formula (III), may be carried out in presence of a suitable base and hydrazine hydrate. The suitable base includes inorganic bases like metallic hydroxides such as but not limited to those of alkali and alkaline-earth metals like calcium, lithium, magnesium, potassium and sodium, or zinc.

The reduction of a compound of Formula (III), may be carried out in presence of a suitable solvent. Suitable solvent may be selected from alcoholic solvents, such as methanol, ethanol, n-propyl alcohol, isopropyl alcohol, n-butanol, the tert-butyl alcohol, triethylene glycol or ethylene glycol.

The addition of base, hydrazine hydrate to the compound of Formula (III) for its reduction may be carried out between 10 to 50°C. More preferably it is between 15 to 45°C. Most preferably it is between 20 to 40°C.

The reduction of a compound of Formula (III), may be carried out at a temperature between 100 to 220°C. more preferably the temperature is between 110 to 210°C. most preferably, it is between 120 to 200°C.

The reduction of a compound of Formula (III), may be carried out for a period of between 2 to 20 hours. More preferably it is between 3 to 15 hours. Most preferably it is from 5 to 12 hours.

The enantioselective oxidation of the compound of Formula (IV), maybe carried out in presence of one or more enzymes to obtain a compound of Formula (V).

The one or more enzymes used for enantioselective oxidation of the compound of Formula (IV) is selected from IEP OX243, IER OX152 or the like and combination thereof.

The aqueous portion of the reaction mixture in which the enantioselective oxidation proceeds preferably contains a buffer, e.g., a dipotassium hydrogen phosphate, potassium phosphate, tris/HCl or triethanolamine buffer.

The enantioselective oxidation of the compound of Formula (IV), may be carried out in a suitable solvent. Suitable solvents may be selected from the group comprising water; ketone, such as acetone, diisobutyl ketone, cyclohexanone, methylcyclohexanone, methyl ethyl ketone, methyl isobutyl ketone, acetylacetone or mixtures thereof.

The enantioselective oxidation of a compound Formula (IV) may be carried out in the presence of a cofactor such as NAD, NADH.

The pH of the reaction mass for the enantioselective oxidation of a compound Formula (IV) may be adjusted using a suitable base such as sodium hydroxide, Sodium bicarbonate (NaHCO₃), Potassium bicarbonate (KHCO₃), Ammonium bicarbonate, Ammonia Solution and the like. Most preferably the base is sodium hydroxide.

The pH of the reaction mass for the enantioselective oxidation of the compound of Formula (IV) may be adjusted between 5 to 10. More preferably, the pH of the reaction is between 6 to 9, most preferably it is between 6.5 to 8.5.

The enantioselective oxidation of the compound of Formula (IV) may be carried out at temperatures between 5 to 40⁰C. More preferably, it is carried out at temperatures between 7.5 to 35°C. Most preferably, it is carried out at temperatures between 10 to 40°C.

The enantioselective oxidation of the compound of Formula (IV) may be carried out for a time period of between 2 to 25 hours. More preferably it is between 4 to 20 hours. Most preferably it is between 5 to 15 hours.

The compound 7-KLCA of Formula (V) obtained from the enantioselective oxidation of the compound Formula (IV) has a HPLC purity of at least 99.5%.

The enantioselective reduction of the compound of Formula (V) is carried out in the presence of one or more enzymes to obtain ursodeoxycholic acid of Formula (I) in a crude form.

The one or more enzymes used for enantioselective oxidation of the compound of Formula (IV) is selected from IEP OX235, IEP SP41 or the like and combination thereof.

The aqueous portion of the reaction mixture in which the enantioselective reduction proceeds preferably contains a buffer, e.g., a dipotassium hydrogen phosphate, potassium phosphate, tris/HCl or triethanolamine buffer.

The enantioselective reduction of the compound of Formula (V), may be carried out in the presence of a suitable solvent. Suitable solvent may be selected from the group comprising water; ketone, such as acetone, diisobutyl ketone, cyclohexanone, methylcyclohexanone, methyl ethyl ketone, methyl isobutyl ketone, acetylacetone and mixtures thereof. The enantioselective reduction of the compound of Formula (V) may be carried out in the presence of a salt of an acid, wherein the salt is a formate salt, and the formate salt is selected from sodium formate and/or ammonium formate.

The enantioselective reduction of compound of Formula (V), may be carried out in presence of a cofactor such as NAD.

The enantioselective reduction of the compound of Formula (V), may be carried at temperatures between 5 to 5°C. More preferably, the temperature is between 10 to 45°C, most preferably, it is between 15 to 35°C.

The enantioselective reduction of the compound of Formula (V), may be carried for a duration of 5 to 45 hours. More preferably, the duration is between 10 to 35 hours. Most preferably the duration is 15 to 25 hours. Ursodeoxycholic Acid of Formula (I) in crude form obtained by the enantioselective reduction of the compound of Formula (V) is obtained in a yield of at least 99% w.r.t to formula (V).

In one embodiment a high-yielding enantioselective enzymatic process is provided for the preparation of Ursodeoxycholic Acid of high enantiomeric purity of at least 99.75 % as measured by HPLC and which is substantially free of the impurities A to H, Hyocholic acid and Hyodeoxycholic acid in an amount between 0.1 to 0.18 % as measured by HPLC, represented by the following chemical structures.

| | |
|---|---|
| Impurity A: Chenodeoxycholic acid | Impurity B: Cholic acid |
| 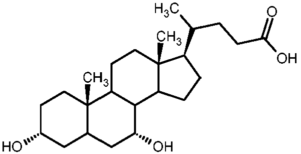 | 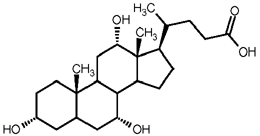 |
| | |
| Impurity C: Lithocholic acid | Impurity D: 3α,7β,12α-trihydroxy-5β-cholan-24-oic acid |
| 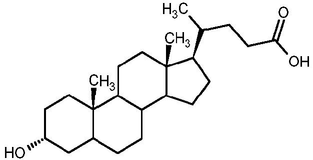 | 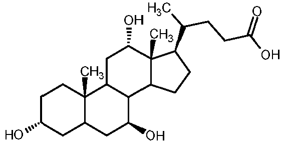 |
| Impurity E: Deoxycholic acid | Impurity F: 7-Ketolithocholic acid |
| 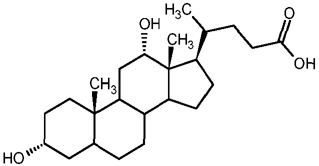 | 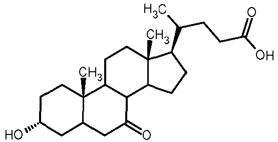 |
| Impurity G: Methyl 3α,7β-dihydroxy-5β-cholan-24-oate | Impurity H: 3β,7β-dihydroxy-5β-cholan-24-oic acid |
| 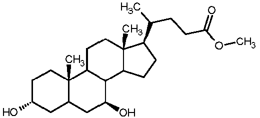 | 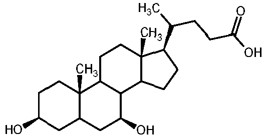 |
| Impurity: Hyocholic acid | Impurity: Hyodeoxycholic acid |
| 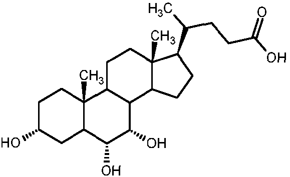 | 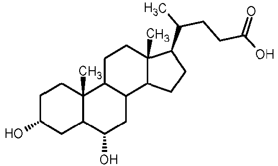 |

and wherein the highly pure Ursodeoxycholic Acid is having chemical purity of about 99.70% to about 99.80% as measured by HPLC, and comprising the impurity A (Chenodeoxycholic acid) in an amount of less than 0.18% and remaining impurities B, C, D, E, F, G, H, HCA and HDCA each one, in an amount of less than 0.1% as measured by HPLC.

In another embodiment a high-yielding enantioselective enzymatic process is provided for the preparation of Ursodeoxycholic Acid of high enantiomeric purity of at least 99.75 % as measured by HPLC and which is substantially free of the impurities B to H, Hyocholic acid and Hyodeoxycholic acid in an amount not more than 0.1 % as measured by HPLCs.

The route of synthesis is schematically represented in Scheme- 1.

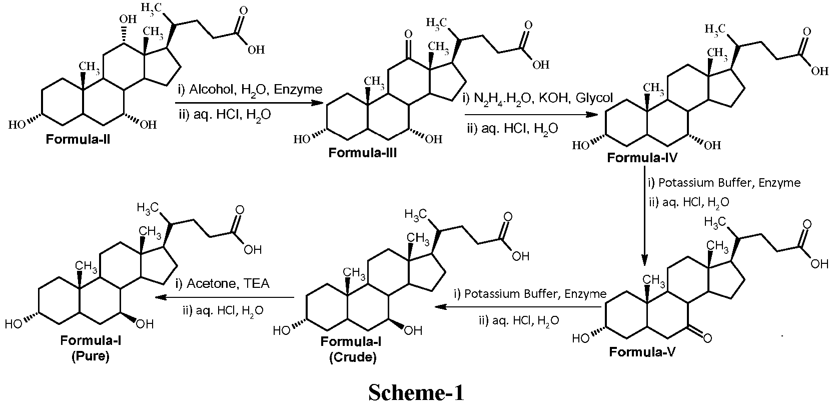

The detailed experimental parameters suitable for making pharmaceutically and enantiomerically pure Ursodeoxycholic Acid are provided by the following examples, which are intended to be illustrative and not limiting to all possible aspects of the invention.

### Example-1: Preparation of compound of Formula (III)

To the solution of dipotassium hydrogen phosphate in water (9V) was charged enzymes IEP OX239 (5%) (sourced from Germany) and IEP LO22 (sourced from Bulgaria) (5%) at 15-40⁰ C under stirring. To the mass were charged Nicotinamide Adenine Dinucleotide (NAD) and cholic acid of formula II (100g), the pH of the mass was adjusted to between 8.5 to 9.5 using aqueous solution of sodium hydroxide. Ethyl alcohol was charged (10g) and stirred the mass for 5-30 hrs. After completion of the reaction, it was heated to 70-75°C and cooled to room temperature. The mass was acidified using aq. HCl between a pH of 1-3. The solids precipitated were filtered, washed with water. The residual enzymes were removed by dissolving the material in methanol (10v). The insoluble mass (enzyme residue) was separated by filtration. The filtrate was concentred to obtain the compound 12-Oxo cholic acid of Formula (III).

**HPLC purity:**12-Oxo cholic acid Purity (97.11%), 7,12-Diketodeoxycholic acid (1.5%), Cholic acid (0.10%), Unspecified impurity (0.32%), total impurities (2.89%).

### Example-2: Preparation of compound of Formula (IV)

To the solution of compound of Formula (III) (100g) in ethylene glycol (5V) was charged aqueous KOH (6eqv) and hydrazine hydrate (3eqv) at 20-40°C under stirring. The reaction mass was heated from 120-200°C. The water was removed by distillation process. After removal of water from the mass, it was maintained at 160-200°C for 5-10hrs. After completion of the reaction, it was cooled to room temperature. The mass was diluted with water and then acidified using aq. HCl. The solids precipitated were filtered, washed with water. The solid obtained was dried under vacuum to obtain the compound CDCA of Formula (IV).

**HPLC purity:** CDCA (97.5%), cholic acid (0.23%), Unspecified impurity (0.12%), total impurities (2.5%).

### Example-3: Preparation of compound of Formula (V)

To the suspension of enzymes IEP OX243 (sourced from Cambrex IEP GmbH, Germany) (5%) and IEP OX152 (sourced from Cambrex IEP GmbH, Germany) (5%) in phosphate buffer(10v) was added NAD at 20-40°C under stirring. After stirring for 30min compound IV (100g) was added. The pH of the mass was adjusted to 6.5-8.5 using aq. Sodium hydroxide solution. Charged acetone (3V) into the mass and reaction mass was stirred at 10-30°C for 5-15 hrs. After completion of the reaction, it was heated to 50-90°C and then cooled to room temperature. The mass was acidified using aq. HCl. The solids precipitated were filtered, washed with water. The solid obtained was dried under vacuum to obtain the compound 7-KLCA of Formula (V). **HPLC purity:**7-KLCA:(99.34%), 7-Ketodeoxycholicacid:(0.07%), Chenodeoxycholic acid: (0.22%), Unspecified impurity: (0.12%), total impurities :(0.66%).

### Example 4: Preparation of compound of Formula (I)

To the suspension of enzymes IEP OX235 (sourced from Cambrex IEP GmbH, Germany) (5%) and IEP SP41 (sourced from Cambrex IEP GmbH, Germany), (10%) in phosphate buffer (2V) (pH 7.0-8.5) was added NAD at 15-35°C under stirring. The suspension was stirred for 30-90min to get a uniform slurry.

The suspension of compound-V (100g) in phosphate buffer (8V) (pH 7.0-8.5) was then charged with the above prepared enzyme slurry (which was prepared in separate reactor) at 15-35°C. Sodium formate, magnesium chloride and Methyl Isobutyl Ketone (MIBK) (2V) were added into the mass and reaction mass was stirred at 15-35°C for 10-30hrs. After completion of the reaction, it was heated to 50-90°C and then cooled to room temperature. The mass was acidified using aq. HCl. The solids precipitated were filtered, washed with water. The solid obtained was dried under vacuum. The dried material was suspended in methanol(10V) and heated to 40-65°C to dissolve the product. The insolubles were removed by filtration. The filtrate was concentrated under vacuum to obtain residue. The residue contains about 0.5% to 1.0% of unreacted 7-Ketolithocholic acid. To the residue was added water (10V) and basified using sodium hydroxide (12g) to get clear solution. The clear solution was then charged with 1g of sodium borohydride and stirred for 8hrs at 25-30°C. After removal of 7-Ketolithocholic acid which was confirmed by HPLC, the reaction mass was acidified using Con. HCl. The solid obtained was filtered. The wet material was dried to obtain Ursodeoxycholic Acid of Formula (I) in crude form. Yield is 98-99%.

**HPLC purity:** UDCA: (97.46%), Chenodeoxycholic acid: (2.26%), Impurity D: (0.01%), Impurity H; (0.17%), Impurity G: (0.01%), Impurity F: (ND), HDCA :(ND), Unspecified impurity: (0.10%), total impurities: (2.54%).

### Example 5: Preparation of compound of Formula (I) in one pot starting from

### compound IV.

To the suspension of enzymes IEP OX243 (sourced from Cambrex IEP GmbH, Germany) (5%) and IEP OX152 (sourced from Cambrex IEP GmbH, Germany) (5%) in phosphate buffer(10v) was added NAD at 20-40°C under stirring. After stirring for 30min compound IV (100g) was added. The pH of the mass was adjusted to 6.5-8.5 using aq. Sodium hydroxide solution. Charged acetone (3V) into the mass and reaction mass was stirred at 10-30°C for 5-15 hrs. After completion of the reaction, it was heated to 50-90°C. Vacuum was applied and acetone distilled out completely from the reaction mass. The reaction mass was cooled to 15-30°C. To the above mass was charged enzymes IEP OX235 (sourced from Cambrex IEP GmbH, Germany) (5%) and IEP SP41 (sourced from Cambrex IEP GmbH, Germany), (10%) in phosphate buffer (5V) (7.0-8.5) was added NAD at 20-40°C under stirring. Charged sodium format, magnesium chloride and Methyl Isobutyl Ketone (MIBK) (2V) into the mass and reaction mass was stirred at 15-35°C for 10-30hrs. After completion of the reaction, it was heated to 50-90°C and then cooled to room temperature. The mass was acidified using aq. HCl. The solids precipitated were filtered, washed with water. The solid obtained was dried under vacuum. The dried material was suspended in methanol(10V) and heated to 40-65°C to dissolve the product. The insolubles were removed by filtration. The filtrate was concentrated under vacuum to obtain residue. The residue contains about 0.5% to 1.0% of unreacted 7-Ketolithocholic acid. Water (10V) was added to the residue and basified using sodium hydroxide (12g) to get a clear solution. The clear solution was charged with 1g of sodium borohydride and stirred for 8hrs at 25-30°C. After removal of 7-Ketolithocholic acid which was confirmed by HPLC, the reaction mass was acidified using Con. HCl. The solid obtained was filtered. The wet material was dried to obtain Ursodeoxycholic Acid of Formula (I) in crude form. Yield is 95-98%.

**HPLC purity:** UDCA: (94.70%), Chenodeoxycholic acid: (2.24%), Impurity D: (0.36%), Impurity H; (0.30%), Impurity G: (0.10%), Impurity F: (ND), HDCA :(0.04%), Unspecified impurity: (0.46%), total impurities: (5.30%).

### Example-6: Preparation of compound of Formula (I)

To the suspension of enzymes IEP OX235 (sourced from Cambrex IEP GmbH, Germany) (5%) and IEP SP41 (sourced from Cambrex IEP GmbH, Germany), (10%) in phosphate buffer (10v) (7.0-8.5) was added NAD at 20-40°C under stirring. After stirring for 30min compound-V (100g) was added. Charged sodium format, magnesium chloride and Methyl Isobutyl Ketone (MIBK) (2V) into the mass and reaction mass was stirred at 15-35°C for 10-30hrs. After completion of the reaction, it was heated to 50-90°C and then cooled to room temperature. The mass was acidified using aq. HCl. The solids precipitated were filtered, washed with water. The solid obtained was dried under vacuum. The dried material was suspended in methanol(10v) and heated to 40-65°C to dissolve the product. The insolubles were removed by filtration. The filtrate was concentrated under vacuum to obtain residue. The residue contains about 0.5% to 1.0% of unreacted 7-Ketolithocholic acid. The residue was charged with water (10V) and basified using sodium hydroxide (12g) to get clear solution. The clear solution was charged with 1g of sodium borohydride and stirred for 8hrs at 25-30°C. After removal of 7-Ketolithocholic acid which was confirmed by HPLC, the reaction mass was acidified using Con. HCl. The solid obtained was filtered. The wet material was dried to obtain Ursodeoxycholic Acid of Formula (I) in crude form. Yield is 98-99%.

**HPLC purity:** UDCA: (97.51%), Chenodeoxycholic acid: (1.73%), Impurity D : (0.1%), Impurity H; (0.18%), Impurity G: (0.06%), Impurity F: (ND), HDCA :(0.06%), Unspecified impurity: (0.29%), total impurities: (2.49%).

### Example 5A: Preparation of compound of Formula (I) using single enzyme IEP 235

To the suspension of enzyme IEP 235 (5%) in phosphate buffer (10V) (7.0-8.5) was added NAD at 20-40°C under stirring. After stirring for 30min compound-V (100g) was added. Charged sodium format, magnesium chloride and Methyl Isobutyl Ketone (MIBK) (2V) into the mass and reaction mass was stirred at 15-35°C for 10-30hrs, reaction was not initiated.

**HPLC data:** 7-KLCA :98.84%, Chenodeoxycholic acid :(0.59%), Unspecified impurity :(0.58%)

### Example 5B: Preparation of compound of Formula (I) using single enzyme IEP SP41

To the suspension of enzymes IEP SP41 (10%) in phosphate buffer (10V) (7.0-8.5) was added NAD at 20-40°C under stirring. After stirring for 30min compound-V (100g) was added. Charged sodium format, magnesium chloride and Methyl Isobutyl Ketone (MIBK) (2V) into the mass and reaction mass was stirred at 15-35°C for 10-30hrs. Reaction was not completed. **HPLC data:** 7- KLCA:97.41%, Chenodeoxycholic acid :(1.58%), Unspecified impurity :(0.95 %)

### Example-6: Purification of compound of Formula (I)

To the suspension of crude ursodeoxycholic acid in acetone (5V) was added triethylamine (1eqv) under stirring. The reaction mass was heated to 40-65°C and stirred for 2-5hrsfollowed by cooling to 10-35°C and filtering the material. The wet material was charged into acetone (5V) and water (2V) mixture, and heated to dissolve completely. The solution was passed through a micron filter and then its pH was adjusted to below 3 using aq. HCl. The mass was diluted with water, stirred for 2-4hrs, and filtered to obtain a white crystalline solid. The material was dried to obtain enantiomerically pure Ursodeoxycholic Acid of formula (I). Yield is 97-99%.

**HPLC purity:** UDCA: (99.75%), Impurity A: (0.18%), Impurity D : (0.01%), Impurity H: (0.02%), Impurity G :(0.01%), Impurity F: (ND), Impurity C :(ND), Impurity: E (ND), Impurity B : (ND), HDCA: (0.01%), HCA :(ND), Unspecified impurity: (0.02%), total impurities :(0.25%).

**Microbial test:** Pseudomonas aeruginosa : absent, Staphylococcus aureus : absent, Total Aerobic Microbial Count :<10 cfu/g, Total Yeast and Mold Count :<10 cfu/g.

### Residual Protein: Below level of quantification (BLQ)

**Table-1: PXRD values of crystalline ursodeoxycholic acid**

| **Peak List** | | | | |
|---|---|---|---|---|
| **Pos. [°2θ]** | **Height [cts]** | **FWHM Left [°2θ]** | **d-spacing [Å]** | **Rel. Int. [%]** |
| 3.8014 | 22345.22 | 0.1732 | 23.22449 | 100.00 |
| 6.6630 | 15647.42 | 0.1082 | 13.25522 | 70.03 |
| 7.4322 | 544.04 | 0.1082 | 11.88497 | 2.43 |
| 9.4084 | 16287.63 | 0.1082 | 9.39255 | 72.89 |
| 11.9707 | 1455.83 | 0.1299 | 7.38726 | 6.52 |
| 13.1091 | 1692.93 | 0.0866 | 6.74820 | 7.58 |
| 13.3197 | 4954.00 | 0.0866 | 6.64197 | 22.17 |
| 13.7266 | 2530.53 | 0.1732 | 6.44596 | 11.32 |
| 14.4029 | 752.11 | 0.0866 | 6.14477 | 3.37 |
| 14.8510 | 5188.73 | 0.1732 | 5.96037 | 23.22 |
| 15.4261 | 1388.69 | 0.0866 | 5.73943 | 6.21 |
| 15.8204 | 1103.79 | 0.1082 | 5.59725 | 4.94 |
| 16.1273 | 1107.24 | 0.0866 | 5.49144 | 4.96 |
| 16.4281 | 1141.76 | 0.1082 | 5.39155 | 5.11 |
| 17.4837 | 1213.55 | 0.1082 | 5.06833 | 5.43 |
| 17.8753 | 1009.16 | 0.3464 | 4.95816 | 4.52 |
| 18.0812 | 1984.18 | 0.1082 | 4.90218 | 8.88 |
| 18.8859 | 3708.95 | 0.0866 | 4.69508 | 16.60 |
| 19.2570 | 928.47 | 0.1948 | 4.60543 | 4.16 |
| 19.6215 | 1289.37 | 0.0866 | 4.52068 | 5.77 |
| 20.0249 | 12443.30 | 0.1082 | 4.43052 | 55.69 |
| 20.2779 | 1205.61 | 0.1299 | 4.37581 | 5.40 |
| 20.7696 | 1402.94 | 0.1299 | 4.27332 | 6.28 |
| 21.1117 | 3711.46 | 0.1082 | 4.20482 | 16.61 |
| 21.3977 | 1078.48 | 0.0649 | 4.14926 | 4.83 |
| 22.4064 | 4237.67 | 0.0866 | 3.96471 | 18.96 |
| 22.8766 | 340.37 | 0.2598 | 3.88427 | 1.52 |
| 24.1152 | 1889.69 | 0.1515 | 3.68749 | 8.46 |
| 24.3418 | 2589.23 | 0.0792 | 3.65367 | 11.59 |
| 24.4381 | 2484.23 | 0.0866 | 3.63950 | 11.12 |
| 24.7338 | 1331.09 | 0.0866 | 3.59665 | 5.96 |
| 25.1630 | 1253.86 | 0.1299 | 3.53628 | 5.61 |
| 25.3568 | 1247.23 | 0.1082 | 3.50968 | 5.58 |
| 26.2293 | 196.38 | 0.2598 | 3.39488 | 0.88 |
| 26.9926 | 578.25 | 0.1299 | 3.30058 | 2.59 |
| 27.6315 | 591.56 | 0.1082 | 3.22571 | 2.65 |
| 28.4934 | 591.43 | 0.1082 | 3.13005 | 2.65 |
| 30.0278 | 131.43 | 0.1299 | 2.97351 | 0.59 |
| 31.7306 | 1927.45 | 0.1515 | 2.81772 | 8.63 |
| 33.2163 | 310.37 | 0.1732 | 2.69500 | 1.39 |
| 33.6652 | 672.39 | 0.0866 | 2.66009 | 3.01 |
| 34.3227 | 432.70 | 0.2165 | 2.61062 | 1.94 |
| 35.0279 | 298.65 | 0.3031 | 2.55966 | 1.34 |
| 36.5014 | 590.19 | 0.0866 | 2.45964 | 2.64 |
| 37.1662 | 377.83 | 0.2598 | 2.41716 | 1.69 |
| 37.7329 | 1470.78 | 0.1082 | 2.38215 | 6.58 |
| 38.4092 | 702.91 | 0.2165 | 2.34174 | 3.15 |
| 38.7998 | 578.01 | 0.1515 | 2.31906 | 2.59 |
| 40.2691 | 411.85 | 0.1732 | 2.23777 | 1.84 |
| 41.2423 | 308.71 | 0.1299 | 2.18719 | 1.38 |
| 41.8401 | 355.71 | 0.1732 | 2.15731 | 1.59 |
| 42.5496 | 406.42 | 0.2598 | 2.12296 | 1.82 |
| 43.8265 | 518.36 | 0.2165 | 2.06403 | 2.32 |
| 44.7706 | 312.66 | 0.1732 | 2.02267 | 1.40 |
| 46.8223 | 229.19 | 0.4330 | 1.93871 | 1.03 |
| 47.8365 | 132.46 | 0.1299 | 1.89994 | 0.59 |
| 48.8370 | 97.68 | 0.2598 | 1.86333 | 0.44 |

## Claims

1. A high-yielding enantioselective enzymatic process for the preparation of ursodeoxycholic acid of Formula (I) with high enantiomeric purity, 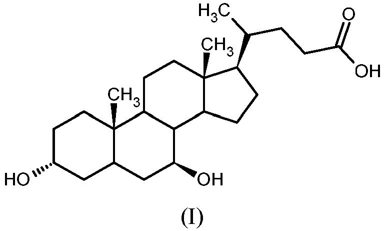 the process comprising the steps of:
a. enantioselectively oxidizing cholic acid of Formula (II); 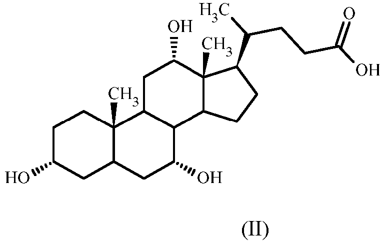 in the presence of an enzyme and a co-factor to obtain an enantiomerically pure compound of Formula (III); 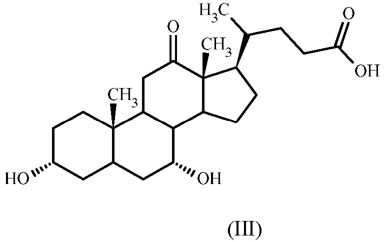
b. reducing the compound of Formula (III) to obtain a compound of formula (IV); 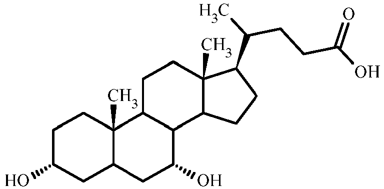 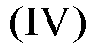
c. enantioselectively oxidising the compound of Formula (IV) in the presence of an enzyme and co-factor to obtain an enantiomerically pure compound of Formula (V); 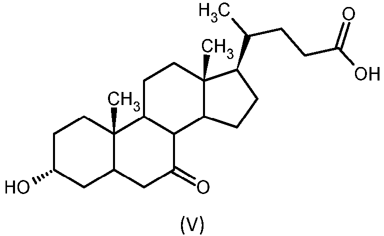
d. enantioselectively reducing the compound of Formula (V) in the presence of an enzyme to obtain crude ursodeoxycholic acid of Formula (I); and
e. treating the crude ursodeoxycholic acid of Formula (I) with a base and an acid to obtain ursodeoxycholic acid of Formula (I) with high enantiomeric purity.

2. The high-yielding enantioselective enzymatic process for the preparation of ursodeoxycholic acid of Formula (I) with high enantiomeric purity of claim 1, wherein the enzyme of step (a) is selected from IEP OX239, IEP LO22P or a combination thereof.

3. The high-yielding enantioselective enzymatic process for the preparation of ursodeoxycholic acid of Formula (I) with high enantiomeric purity of claim 1, wherein the co-factor is selected from NAD or NADH.

4. The high-yielding enantioselective enzymatic process for the preparation of ursodeoxycholic acid of Formula (I) with high enantiomeric purity of claim 1, wherein the reduction of step (b) is carried out in the presence of a base and reducing agent, wherein the reducing agent is selected from sodium borohydride, potassium borohydride, lithium borohydride, sodium cyanoborohydride, triacetoxy sodium borohydride, and wherein the base is selected from sodium hydroxide, potassium hydroxide, lithium hydroxide, sodium bicarbonate, potassium carbonate, potassium bicarbonate, sodium carbonate.

5. The high-yielding enantioselective enzymatic process for the preparation of ursodeoxycholic acid of Formula (I) with high enantiomeric purity of claim 1, wherein the enzyme of step (c) is selected from IEP OX243 and IEP OX152 or combination thereof.

6. The high-yielding enantioselective enzymatic process for the preparation of ursodeoxycholic acid of Formula (I) with high enantiomeric purity of claim 1, wherein the enzyme of step (d) is a combination of IEP 235 and IEP SP41.

7. The high-yielding enantioselective enzymatic process for the preparation of ursodeoxycholic acid of Formula (I) with high enantiomeric purity of claim 1, wherein the yield of crude ursodeoxycholic acid of Formula (I) of step (d) is at least 99%.

8. The high-yielding enantioselective enzymatic process for the preparation of ursodeoxycholic acid of Formula (I) with high enantiomeric purity of claim 1, wherein the treatment of crude ursodeoxycholic acid of Formula (I) of step (e) is carried out sequentially.

9. The high-yielding enantioselective enzymatic process for the preparation of ursodeoxycholic acid of Formula (I) with high enantiomeric purity of claim 1, wherein the ursodeoxycholic acid obtained is at least 99.75% enantiomerically pure and wherein the ursodeoxycholic acid is substantially free of impurities A to H, Hyocholic acid and Hyodeoxycholic acid.

10. The high-yielding enantioselective enzymatic process for the preparation of ursodeoxycholic acid of Formula (I) with high enantiomeric purity of claim 1, wherein the ursodeoxycholic acid of high enantiomeric purity of step (e) is devoid of Impurity B, Impurity C, Impurity E, Impurity F, and impurity Hyocholic acid as measured by HPLC.

11. The high-yielding enantioselective enzymatic process for the preparation of ursodeoxycholic acid of claim 1, wherein the highly pure ursodeoxycholic acid obtained from step (e) has a chemical purity of between 99.70% to 99.80% as measured by HPLC, wherein the impurity A is present in an amount of less than 0.18%, and wherein impurities B, C, D, E, F, G, H, impurity HCA and impurity HDCA are independently present in an amount of less than 0.1% as measured by HPLC

12. An enantioselective enzymatic process for the preparation of a compound of Formula (V) with high enantiomeric purity, useful for the preparation of ursodeoxycholic acid of Formula (I) with high enantiomeric purity, 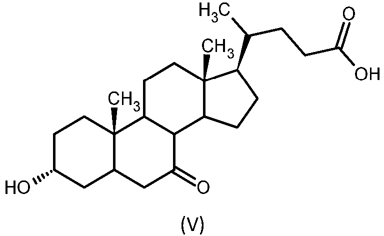 the process comprising the steps of:
enantioselectively oxidizing a compound of Formula (IV) 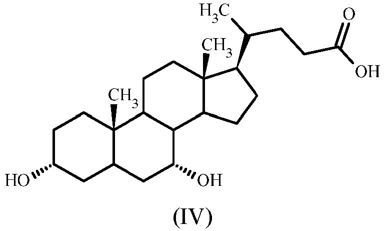
in the presence of an enzyme and co-factor to obtain an enantiomerically pure compound of Formula (V);
optionally wherein the enantiomeric purity of the compound of Formula (V) is at least 99.75%.

13. A high-yielding enantioselective enzymatic process for the preparation of crude ursodeoxycholic acid of Formula (I), 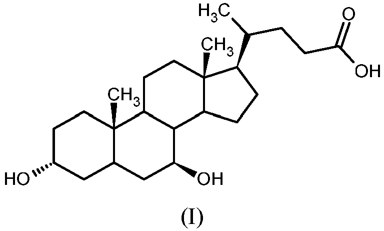 the process comprising the step of enantioselectively reducing a compound of Formula (V) in the presence of suitable enzymes to obtain crude ursodeoxycholic acid of Formula (I).

14. A purification process for the preparation of highly pure ursodeoxycholic acid compound of Formula (I), 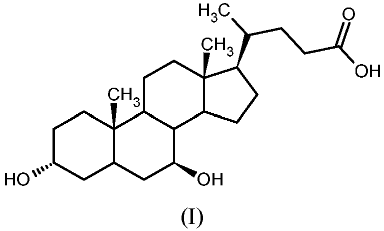 the process comprising the steps of:
(a) dissolving crude ursodeoxycholic acid in acetone to produce a reaction mass,
(b) heating the reaction mass at a suitable temperature and stirring for 2-5 hours,
(c) cooling the reaction mass to room temperature and filtering any resulting solid,
(d) completely dissolving the filtered solid in acetone and water mixture,
(e) adjusting the pH below 3 with a suitable acid to obtain a mass,
(f) diluting the mass with water and stirring for 2-4 hours; and
(g) collecting the highly pure ursodeoxycholic acid by filtration as a white crystalline solid;
optionally wherein step (a) further comprises addition of trimethylamine base.

15. The purification process for the preparation of highly pure ursodeoxycholic acid of claim 14, wherein the suitable temperature of heating of step (c) is between 30-75°C, and wherein the suitable acid of step (f) is selected from hydrochloric acid, hydrobromic acid, nitric acid, sulfuric acid, acetic acid, trifluoroacetic acid.
